# EUROPEAN PATENT APPLICATION

(11) **EP 1 925 313 A1**
(43) Date of publication of application: **28.05.2008**
(21) Application number: 06124781.3
(22) Date of filing: 24.11.2006
(51) Int. Cl.: A61K 36/53

(54) **Prevention and treatment of disorders connected to impaired neurotransmission**

(71) Applicant: Nestec S.A., 1800 Vevey (CH); DSM IP Assets B.V., 6411 TE Heerlen (NL)
(72) Inventor: The designation of the inventor has not yet been filed
(74) Representative: Dixon, Sarah

(57) **Abstract**

The present invention relates to the use of rosemary extracts for the prevention and treatment of disorders connected to impaired neurotransmission such as depression in humans, companion animals and farm animals

## Description

The present invention relates to the use of rosemary extracts for the prevention and treatment of disorders connected to impaired neurotransmission.

### Background of the Invention

It is well known that mental disorders such as depression are related to impaired neurotransmission, e.g. low neurotransmitter levels.

Compounds that increase neurotransmitter levels in the brain and thus enhance their transmission therefore exhibit antidepressant properties as well as beneficial effects on a variety of other mental disorders ("Neurotransmitters, drugs and brain function" R.A. Webster (ed), John Wiley & Sons, New York, 2001, p. 187-211, 289-452, 477-498). The main neurotransmitters are serotonin, dopamine, norepinephrine, noradrenaline, acetylcholine, glutamate, gamma-amino-butyric acid, as well as neuropeptides.
Increase in neurotransmission is achieved by increasing the concentration of the neurotransmitter in the synaptic cleft thus making it available for increased or prolonged neurotransmission through inhibition of re-uptake into the pre-synaptic nerve end, or by preventing neurotransmitter catabolism by inhibition of degrading enzymes such as monoaminooxidase A and B.

Tricyclic antidepressant compounds (TCAs) such as imipramine, amitriptyline, and clomipramine for example inhibit the re-uptake of serotonin and noradrenaline. They are widely regarded as among the most effective antidepressants available, but they have a number of disadvantages because they interact with a number of brain receptors, e.g. with cholinergic receptors. Most importantly, there are risks associated with overdoses of TCAs including acute cardiotoxicity.

Another class of antidepressant drugs are the so-called SSRIs (selective serotonin re-uptake inhibitors) including fluoxetine, paroxetine, sertraline, citalopram and fluvoxamine which block the serotonin transporter (SERT), a high affinity sodium chloride-dependent neurotransmitter transporter that terminates serotonergic neurotransmission by uptake of serotonin, thus inhibiting re-uptake of serotonin alone. They have been demonstrated to be effective in the treatment of depression and anxiety and are usually better tolerated than TCAs. These medications are typically started at low dosages and dosage level may be increased until they reach a therapeutic level. A common side effect is nausea. Other possible side effects include decreased appetite, dry mouth, sweating, infection, constipation, yawning, tremors, sleepiness and sexual dysfunction.

In addition, compounds that prevent the catabolism of neurotransmitters more broadly by inhibiting the monoaminooxidases (MAOs) A and B exhibit antidepressant effects. MAOs catalyse the oxidation of amino group containing neurotransmitters such as serotonin, noradrenaline, and dopamine.

Furthermore, modulators of neurotransmission exert pleiotropic effects on mental and cognitive functions.

There is a need for compounds for the treatment or prevention of mental diseases and/or disorders which do not show the negative side effects of known antidepressants. Many patients are interested in alternative therapies which could minimize the side effects associated with high doses of drugs and yield additive clinical benefits. Severe depression is a long lasting and recurring disease, which is usually poorly diagnosed. Furthermore many patients suffer from mild or moderately severe depression. Thus, there is an increasing interest in the development of compounds as well as pharmaceutical and/or dietary compositions that may be used to treat mental diseases/disorders or to prevent the development of mental diseases/disorders such as depression in people at risk, and to stabilize mood.

### Summary of the Invention

Remarkably it has now been found that extracts of the plant *Rosmarinum officinalis* (common name rosemary) may be used in the management of diseases and disorders related to impaired neurotransmission.

Accordingly, the present invention provides the use of an extract of *Rosmarinum officinalis* in the manufacture of a medicament or nutritional composition for the prevention and/or treatment of a disease or disorder associated with impaired neurotransmission in a mammal.

The invention further extends to a method of preventing and/or treating a disease or disorder associated impaired neurotransmission in a mammal comprising administering to a mammal in need thereof a therapeutic amount of an extract of *Rosmarinum officinalis.*

The disease or disorder associated with impaired neurotransmission may be, for example, depression, bulimia or obsessive compulsive disorder. Further the extract may also be used as a mood improver, a stress reliever or a condition improver.

The mammal may be a human or an animal, for example a companion animal such as a dog or cat.

### Detailed Description of the Invention

In the following description:-
The expression "rosemary extract" is used to denote an extract of *Rosmarinum officinalis.* The rosemary extract may be from any part of the plant e.g. leaves, seeds, roots, embryos or cell cultures. The extract may be in the form of a dried, lyophilized extract of leaves, roots and/or seeds or may be an enriched fraction obtained by an inorganic or organic solvent extraction process known in the art
The word "disorder" also encompasses diseases.

The word "prevention" includes prevention of both a first occurrence of a disorder (primary prevention) and prevention of a re-occurence (secondary prevention).

According to the present invention not only the rosemary extracts themselves, but also nutritional and pharmaceutical compositions containing them can be used for the treatment of disorders and diseases related to impaired neurotransmission.

The rosemary extract may be prepared by any suitable method known in the art having due regard for the nature of the product in which it is to be incorporated. For example, a food grade rosemary extract may be prepared by drying, for example freeze drying, the leaves of the rosemary plant. The dried leaves may then by extracted using a suitable solvent, for example hexane or a mixture of hexane and ethanol, preferably under an inert atmosphere. The liquid phase may then be separated from the solid phase by vacuum filtration. The filtrate may be reserved and the solid phase subjected a second extraction similar to the first following which the two filtrates may be mixed and concentrated as desired, for example by fractionation.

Alternatively, the rosemary extract may be prepared by defatting ground dried leaves with hexane, subjecting the defatted material to an acidic hydrolysis and then extracting the aqueous hydrolysed phase with a non-miscible solvent such as ethyl acetate. Again, the resulting extract may be fractionated if desired.

The rosemary extract may be used in the preparation of a medicament or a nutritional composition for humans or animals.

In one embodiment, the nutritional composition is a food composition for human consumption. This composition may be a nutritionally complete formula, a dairy product, a chilled or shelf stable beverage, a soup, a dietary supplement, a meal replacement, or a nutritional bar for example.

A nutritionally complete formula according to the invention may comprise a source of protein. Any suitable dietary protein may be used for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred. The composition may also contain a source of carbohydrates and a source of fat.

If the formula includes a fat source, the fat source preferably provides 5% to 40% of the energy of the formula; for example 20% to 30% of the energy. A high intake of n-6 polyunsaturated fats should preferably be avoided where there is a risk of stress and inflammatory conditions. Preferably, the ratio of n6/n3 fatty acids should be between 2:1 and 6:1. These requirements can be met by using a blend of canola oil, corn oil and high-oleic acid sunflower oil supplemented with a source rich in n3 PUFA such as fish oil if a higher n3 content is required.

A source of carbohydrate may be added to the formula. It preferably provides 40% to 80% of the energy of the formula. Any suitable carbohydrate may be used, for example sucrose, lactose, glucose, fructose, corn syrup solids, maltodextrins, and mixtures thereof. Dietary fibre may also be added if desired. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, gum Arabic, fructooligosaccharides, galacto-oligosaccharides, sialyl-lactose and oligosaccharides derived from animal milks. A preferred fibre blend is a mixture of outer pea fibre (predominantly insoluble), acacia gum and short chain fructo-oligosaccharides (both soluble). Preferably, if fibre is present, the fibre content is between 10 and 40 g/l of the formula as consumed.

The formula may also contain minerals, micronutrients and trace elements in accordance with the recommendations of Government bodies such as the USRDA

One or more food grade emulsifiers may be incorporated into the formula if desired; for example diacetyl tartaric acid esters of mono- and di- glycerides, lecithin and mono- and di-glycerides. Similarly suitable salts and stabilisers may be included.

The formula is preferably enterally administrable; for example in the form of a powder or a liquid concentrate for re-constitution with milk or water, a solid product or a ready-to-drink beverage.

The formula may be prepared in any suitable manner. For example, it may be prepared by blending together the protein, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture. The liquid mixture is then homogenised; for example in two stages.

The liquid mixture may then be thermally treated to reduce bacterial loads, by rapidly heating the liquid mixture to a temperature in the range of about 80°C to about 150°C for about 5 seconds to about 5 minutes, for example. This may be carried out by steam injection, autoclave or by heat exchanger; for example a plate heat exchanger.

Then, the liquid mixture may be cooled to about 60°C to about 85°C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

If it is desired to produce a powdered formula, the homogenised mixture is transferred to a suitable drying apparatus such as a spray drier or freeze drier and converted to powder. The powder should have a moisture content of less than about 5% by weight.

If it is desired to produce a liquid formula, the homogenised mixture is preferably aseptically filled into suitable containers by pre-heating the homogenised mixture (for example to about 75 to 85°C) and then injecting steam into the homogenised mixture to raise the temperature to about 140 to 160°C; for example at about 150°C. The homogenised mixture may then be cooled, for example by flash cooling, to a temperature of about 75 to 85°C. The homogenised mixture may then be homogenised, further cooled to about room temperature and filled into containers. Suitable apparatus for carrying out aseptic filling of this nature is commercially available. The liquid composition may be in the form of a ready to feed formula having a solids content of about 10 to about 14% by weight or may be in the form of a concentrate; usually of solids content of about 20 to about 26% by weight.

In another embodiment, a conventional food product such as a yoghurt, or a breakfast cereal may be enriched with the rosemary extract.

In a further embodiment, the rosemary extract may be used in the preparation of a pet food composition. The said composition may be administered to the pet as a supplement to its normal diet or as a component of a nutritionally complete pet food, or in a hypocaloric pet food.

A nutritionally complete pet food composition according to the invention may be in powdered, dried form, a treat or a wet, chilled or shelf stable pet food product. These pet foods may be produced by methods known in the art.

The pet food may optionally also contain a prebiotic, a probiotic microorganism or another active agent, for example a long chain fatty acid. If present, the amount of prebiotic in the pet food is preferably less than 10% by weight. For example, the prebiotic may comprise about 0.1% to about 5% by weight of the pet food. For pet foods which use chicory as the source of the prebiotic, the chicory may be included to comprise about 0.5% to about 10% by weight of the feed mixture; more preferably about 1% to about 5% by weight.

If a probiotic micro-organism is used, the pet food preferably contains about 10⁴ to about 10¹⁰ colony forming units (cfu) of the probiotic micro-organism per gram of the pet food; more preferably about 10⁶ to about 10⁸ cfu per gram.

If necessary, the pet food may be supplemented with minerals and vitamins. Further, various other ingredients, for example, sugar, salt, spices, seasonings, flavouring agents, and the like may also be incorporated into the pet food as desired.

In another embodiment, dietary adjuncts may be prepared so as to improve pet food quality. As dietary adjuncts, they may be encapsulated or may be provided in powder form and packaged in conjunction with or separately from a main meal, be it wet or dry. By way of example, a powder containing extracts according to the invention, may be packed in sachets in a powder form or in a gel or lipid or other suitable carrier. These separately packaged units may be provided together with a main meal or in multi-unit packs for use with a main meal or treat, according to user instructions.

In yet a further embodiment, a medicament containing a rosemary extract as described above in an amount sufficient to achieve the desired effect in an individual can be prepared. This medicament may be in the form of tablets, capsules, pastilles or a liquid for example. The medicament, which may be for humans or animals, may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The medicament may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. Further, the medicament may contain an organic or inorganic carrier material suitable for oral or enteral administration.

Disorders associated with impaired neurotransmission include unipolar depression, bipolar depression, acute depression, chronic depression, sub-chronic depression, dysthymia, postpartum depression, premenstrual dysphoria/syndrom (PMS), climacteric depressive symptoms, aggression, attention deficit disorders (ADS), social anxiety disorders, seasonal affective disorders, anxiety (disorders), fibromyalgia syndrome, chronic fatigue, sleep disorders (insomnia), post-traumatic stress disorders, panic disorders, obsessive compulsive disorders, restless leg syndrome, nervousness, migraine/primary head-aches and pain in general, emesis, bulimia, anorexia nervosa, binge eating disorder, gastrointestinal disorders, burn out syndrome, irritability and tiredness.

Rosemary extracts may also be used in the manufacture of compositions for the primary and secondary prevention and/or treatment of neurocognitive impairment such as mild cognitive impairment in the elderly, the treatment of depressive symptoms or other symptoms related to disturbed neurotransmission occurring as co-morbidity in chronic diseases such as cardio-vascular diseases, strokes, cancer, Alzheimer disease, Parkinson disease, and others.

In certain circumstances, pet animals and farm animals may also be in need of enhanced or improved neurotransmission. Such circumstances may occur after capture, during or after transport, or following rehousing or relocation, when the animals may develop analogous disorders and become distressed or aggressive.

Thus, rosemary extract may be used in general as antidepressants for animals including humans, preferably for humans, companion animals and farm animals.

In a further embodiment of the present invention rosemary extracts find use in the manufacture of medicaments and nutritional compositions for use as mood improvers. "Mood improver " means that the mood of a person treated is enhanced, that the self esteem is increased and/or that negative thoughts are reduced, emotions are balanced and/or general well being is improved.

Moreover, rosemary extracts may be used in the manufacture of medicaments and nutritional compositions for the treatment and prevention of stress, the alleviation of stress related symptoms, the increase of resistance or tolerance to stress and/or to favour and facilitate relaxation in normal healthy individuals i.e. such medicaments and compositions have an effect as a "stress reliever".

A further embodiment of the present invention relates to the use of rosemary in the manufacture of medicaments and nutritional compositions for use as "condition improvers", i.e. to reduce irritability and tiredness, to favour undisturbed sleep, for the regulation of hunger and satiety as well as for the regulation of motor activity and to increase energy in more general terms in diseased or normal healthy individuals.

For both humans and animals, a suitable daily dosage of rosemary extract for the purposes of the present invention may be within the range from 1 mg per kg body weight to about 100 mg per kg body weight per day. More preferred is a daily dosage of about 3 to about 30 mg per kg body weight, and especially preferred is a daily dosage of about 10 to 20 mg per kg body weight.

In medicaments for humans and animals, the rosemary extract as defined is suitably present in an amount from about 1 mg to about 1000 mg, preferably from about 200 mg to about 500 mg per dosage unit.

The invention is illustrated further by the following examples.

### Example 1: Preparation of the rosemary extract

40g of rosemary leaves were defatted with 600ml hexane. The mixture was filtered to remove the hexane, concentrated, dried and ground into powder. 300ml of 1M hydrochloric acid was added to the powder to effect an acid hydrolysis and the aqueous hydrolysate was extracted with three separate aliquots of 150ml of ethyl acetate. The extract was filtered and concentrated and dried over sodium sulphate.

100 mg of the extract were dissolved in 3 ml methanol (Merck), centrifuged and filtered (0.2 µm). 2 ml of this solution (c = 33 mg/ml) were fractionated via chromatography (reversed phase RP18, water/methanol/acetonitrile (plus ammonium acetate buffer) gradient) in 59 fractions.

### Example 2: Serotonin uptake inhibition by rosemary extract.

HEK-293 cells stably expressing the **h**uman **s**erotonin **r**e-uptake **t**ransporter (hSERT) were obtained from R. Blakely, Vanderbilt University, USA. The cells were routinely grown in Dulbeco's Modified Eagles Medium (Bioconcept) containing 10% fetal calf serum, penicillin, streptomycin, L-glutamine and the antibiotic G418 and passaged by trypsinisation. On the day of assay, cells from 80% confluent flasks were harvested by gentle washing with warm phosphate buffered saline (PBS). Cells were then washed once by centrifugation and re-suspended in Krebs Ringers bicarbonate buffer (Sigma) supplemented with 35 µM pargyline, 2.2 mM CaCl₂, 1 mM ascorbic acid and 5 mM N-2-**h**ydroxyethyl**p**iperazine-N'-2-**e**thanesulfonic acid (buffer called "Hepes") at a concentration of 10,000 cells in 160 ul of buffer, and aliquoted into round bottomed polypropylene 96 well microtitre plates (Corning) at 10,000 cells per well. Serotonin uptake into the cells was determined by addition of radio-labeled (3H) serotonin (GE Healthcare) to a concentration of 20 nM, and incubation for 40 minutes at 37°C with gentle shaking. At the end of this time unincorporated label was removed by filtration though Unifilter 96 GF/B plates (Perkin Elmer) using a Tomtec Mach III M cell harvester. The incorporated serotonin retained on the plates was quantified by liquid scintillation counting using Microscint-40 / Topcount (Perkin Elmer).

Membranes containing hSERT were prepared from the above cell line according to Galli et al, Journal of Experimental Biology 1995, 198, 2197-2212. 3 µg of membrane protein and 0.8 mg of wheat germ aglutinin coated SPA® (**s**cintillation **p**roximity assay) beads (GE Healthcare) per data point were mixed with radio-labeled (3H) citalopram (GE Healthcare) until a final concentration of 3.3 nM in 50mM Tris-HCl, 300 mM NaCl (pH 7.4). After incubation for 18 hours at room temperature, the bound citalopram was quantified by scintillation counting using the Topcount (Perkin Elmer).

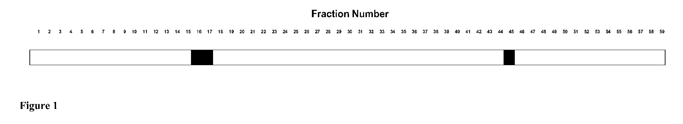

The effect of the rosemary extract on the serotonin uptake was determined by their inclusion in the assay at a range of concentrations between 0.03 and 100 µM for 10 minutes prior to and during the addition of (3H) serotonin. Figure 1 shows the inhibitory activity of the fractionated extracts on (3H) serotonin uptake.

**Figure 1:** Comparison of serotonin uptake inhibition versus fraction number for fractionated ethyl acetate extract of rosemary leaves. The black areas denote fractions which caused greater than 25% inhibition of (3H) serotonin uptake relative to control (0.25% DMSO alone). The actual values determined were 61% for fraction 16, 62% for fraction 17 and 100% for fraction 45.

## Claims

1. Use of an extract of *Rosmarinum officinalis* in the manufacture of a medicament or nutritional composition for the prevention or treatment of a disease or disorder associated with impaired neurotransmission in a mammal.

2. The use of Claim 1, wherein the disorder is neurocognitive impairment, unipolar depression, bipolar depression, acute depression, chronic depression, sub-chronic depression, dysthymia, postpartum depression.

3. Use of an extract of *Rosmarinum officinalis* in the manufacture of a medicament or nutritional composition which is a mood improver in a mammal.

4. Use of an extract of *Rosmarinum officinalis* in the manufacture of a medicament or nutritional composition which is a stress reliever in a mammal.

5. The use of Claims 1 to 4, wherein the mammal is a human.

6. The use of Claims 1 to 4, wherein the mammal is a companion or farm animal.

7. The use of any preceding claim, wherein the extract is obtained from the leaves, seeds, roots, embryos or cell cultures of *Rosmarinum officinalis.*

8. The use of any preceding claim in the manufacture of a medicament comprising from 200 to 500mg of the extract per dosage unit.

9. The use of Claims 1 to 8, wherein the nutritional composition comprises a source of protein, a source of carbohydrate and a source of lipids.
